# EUROPEAN PATENT SPECIFICATION

(11) **EP 3 946 071 B1**
(45) Date of publication and mention of the grant of the patent: **04.06.2025**
(21) Application number: 20795303.5
(22) Date of filing: 24.04.2020
(51) Int. Cl.: A61B 17/00, A61B 17/06, A61B 17/04, A61B 17/062

(54) **SUTURE SECURITY DEVICE FOR MINIMALLY INVASIVE SURGICAL SUTURING**
NAHTSICHERHEITSVORRICHTUNG FÜR MINIMALINVASIVES CHIRURGISCHES NÄHEN
DISPOSITIF DE SÉCURITÉ DE SUTURE POUR SUTURES CHIRURGICALES MINIMALEMENT INVASIVES

(30) Priority: 24.04.2019 US 201962838281 P
(43) Date of publication of application: 09.02.2022
(73) Proprietor: LSI Solutions, Inc., Victor, NY 14564 (US)
(72) Inventor: SAUER, Jude, S., Pittsford, NY 14534 (US)
(74) Representative: Flügel Preissner Schober Seidel
(86) International application number: PCT/US2020/029688
(87) International publication number: WO 2020/219791

(56) References cited:
- EP-A1- 0 609 020
- WO-A1-00/19913
- WO-A1-2018/075568
- WO-A1-2020/251986
- WO-A1-96/25102
- WO-A2-2007/138284
- US-A- 5 100 421
- US-A- 6 004 332
- US-A1- 2006 069 397
- US-A1- 2008 091 220
- US-A1- 2015 190 132
- US-A1- 2016 151 064
- US-B1- 9 079 006

## Description

### FIELD

The claimed invention relates generally to surgical instruments for automated suturing, and more particularly to such a surgical instrument having a suture security device to ensure sutures are not deployed prematurely.

### BACKGROUND

Surgical instruments for automated suturing, such as the RD180^{®} device from LSI Solutions, Inc., Victor, NY (www.lsisolutions.com) provide a reliable and effective way to deliver a suture stitch remotely through a minimally invasive surgical opening. Such devices typically have a suture loaded therein, whereby the suture has a metal tube or ferrule attached to the end of the suture. The ferrule sits in a ferrule holder on one side of a tissue gap on the distal end of the suturing device. In axial alignment with the held ferrule is a needle on the opposite side of the device's tissue gap. The device is oriented to place a targeted tissue location within the tissue gap, and a needle actuator is engaged to move the needle through the tissue in the gap, into interference contact with the ferrule, causing the ferrule to couple with the needle. The needle actuator is released, thereby causing the needle to retract back through the tissue in the tissue gap, also pulling the suture attached to the ferrule back through the tissue and this creating a stitch in the tissue.

In general, ferrule holders are very effective for holding the ferrules in alignment with the needle of the device until a stitch is made. However, automated suturing devices continue to be developed for ever-expanding applications, the environment within which the device may be used and/or the flexibility and steerability of the device shaft which is needed to position the tissue gap onto target tissue may cause the ferrule held in the ferrule holder to come loose prior to when the needle is actuated. If this happens, the suturing device may not work as intended.

One example of a suture security device is disclosed by WO 2020/251986 A1 which is a prior art document according to Art. 54(3) EPC. Therein is a suture security device disclosed comprising a latch which is moved directly by an actuator up and down towards a plate to hold a suture therebetween.

US 9 079 006 B1 discloses a medical catheters anchored within a body cavity or outside the body by restraining a flexible filament or suture comprising a spring finger and a spring clamp.

WO 2007 / 138284 A2 discloses a device for assisting in the tying of knots in a length of suture and WO 00/19913 A1 discloses a laparoscopic surgical instrument.

US 6 004 332 A discloses an apparatus for suturing anatomical tissue during endoscopic and open surgical procedures. The apparatus includes jaw members that are configured to grasp a suture therebetween.

Therefore, there is a need for a suture security device for such automated suturing devices so have a high assurance for certain suturing applications that the ferrule remains in its ferrule holder until the needle is ready to pick it up.

### SUMMARY

The invention is set out in the appended set of claims.

A suture security device for automated surgical suturing is disclosed. The suture security device also includes a latch comprising a cam linkage and being pivotable about an axis, a plate, and an actuator coupled to the latch by the cam linkage and operable to move the latch away from the plate. The latch is further configured to be selectively held towards the plate, thereby being configured to grip a suture therebetween.

Another suture security device for automated surgical suturing is also disclosed. The suture security device includes a first latch array, a second latch array, a plurality of plates, a first actuator coupled to the first latch array by a first cam linkage and operable to move the first latch array away from the plurality of plates, and a second actuator coupled to the second latch array by a second cam linkage and operable to move the second latch array away from the plurality of plates. The first latch array and the second latch array comprise each a plurality of latches and are each pivotable about an axis. The first latch array comprise the first cam linkage and the second latch array comprises the second cam linkage. The first latch array and the second latch array are configured to be selectively held towards the plurality of plates, thereby being configured to grip a suture therebetween.

An additional suture security device is also disclosed. The suture security device also includes a housing, a first latch array having a plurality of latches coupled to the housing, a second latch array having a plurality of latches coupled to the housing and interlaced with the first latch array, a plurality of plates, a first actuator coupled to the first latch array and operable to move the first latch array away from the plurality of plates, and a second actuator coupled to the second latch array and operable to move the second latch array away from the plurality of plates.

### BRIEF DESCRIPTION OF THE DRAWINGS

FIGS. 1A-1C are schematic illustrations of one embodiment of a suture security device 20 for surgical suturing.
FIGS. 2A-2J are exploded views illustrating assembly of another embodiment of a suture security device.
FIG. 3 is a partially exposed view of the assembly of the suture security device

It will be appreciated that for purposes of clarity and where deemed appropriate, reference numerals have been repeated in the figures to indicate corresponding features, and that the various elements in the drawings have not necessarily been drawn to scale in order to better show the features.

### DETAILED DESCRIPTION

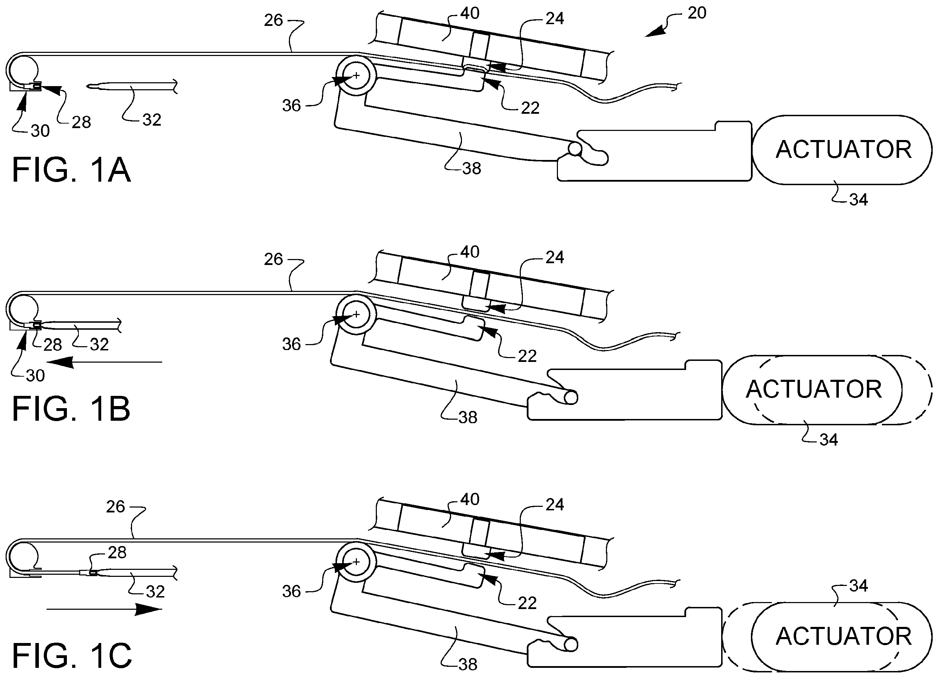

FIG. 1A is a schematic illustration of one embodiment of a suture security device 20 for surgical suturing. The device has a latch 22 which may be selectively held towards a plate 24. A suture 26 may be placed between the latch 22 and its plate 24. At least one end of the suture 26 has a ferrule 28 coupled to it. The ferrule 28 is placed into a ferrule holder 30 and the suture 26 is routed back towards the latch 22 and plate 24. The suture 26 may be lightly tensioned or otherwise adjusted for length so that the ferrule 28 is held in the ferrule holder 30. The latch 22 may be engaged or held towards the plate 24, gripping the suture 26 therebetween in order to maintain the suture position and provide additional insurance that the ferrule 28 will not prematurely leave the ferrule holder 30. As described above in the background, a needle 32 is axially aligned opposite from the loaded ferrule 28.

As schematically illustrated in FIG. 1B, the needle 32 may be actuated towards and into contact with the ferrule 28. At approximately the same time, or any desired relative time, an actuator 34 may also be engaged to move the latch 22 away from the plate 24 in order to release the held suture 26. At the point of FIG. 1B, the needle 32 has properly engaged the ferrule 28, so there is no need to secure the ferrule 28 in the ferrule holder 30 at this time. By releasing the latch 22's grip on the suture 26, the needle 32 is then free to retract as shown in FIG. 1C, pulling the ferrule 28 and its attached suture 26 back across a tissue gap.

In the embodiment of FIGS. 1A-1B, the actuator 34 which moves the latch 22 away from the plate 24 may be coupled to the actuator for the needle 32, or it may be separately actuated. In the embodiment shown, the latch 22 is pivotable about an axis 36 as moved by a cam linkage 38 coupled to the actuator 34. In this way, the suture 26 is secured until it needs to be released. For some embodiments, the plate 24 may be part of, supported by, or adjacent to a window 40 through which a user can view the held suture 26 and see when it has moved after ferrule pickup in order to remotely verify a successful needle pickup since the distal tip with the needle 32 and ferrule 28 may not be visible to the user.

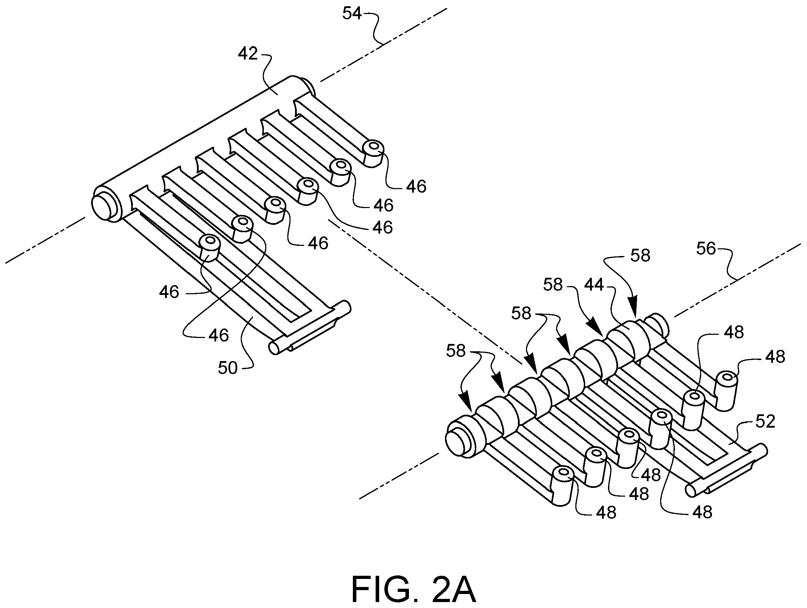

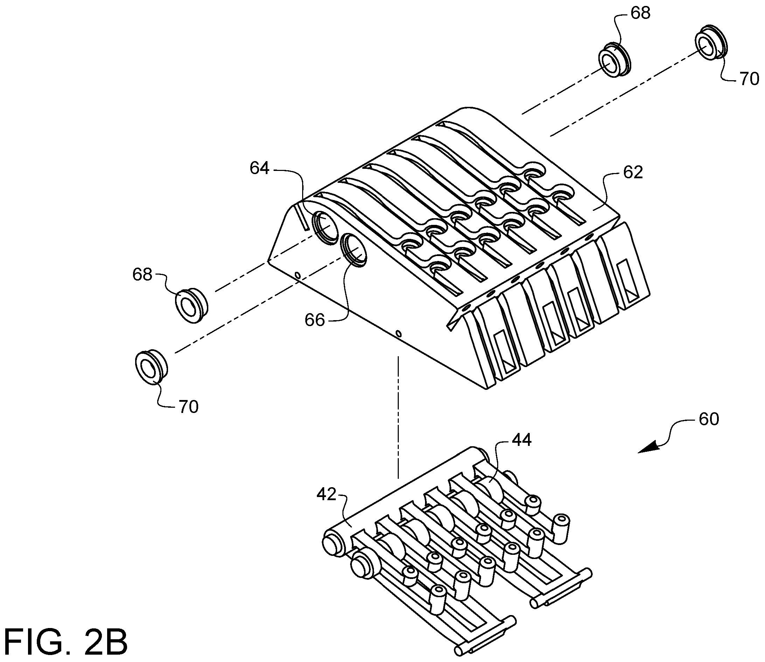

FIGS. 2A-2J are exploded views illustrating assembly of another embodiment of a suture security device. This device has a plurality of latches, represented by a first latch array 42 and a second latch array 44. The first latch array 42 has a plurality of latches 46, while the second latch array has a plurality of latches 48. In this embodiment, each latch array 42, 44 has six latches 46, 48, respectively, meaning the final unit will be able to secure twelve sutures, six with the first latch array 42 and six with the second latch array 44. Each latch array 42, 44 has its own respective cam linkage 50, 52. Similarly, each latch array 42, 44 has its own respective pivot axis 54, 56. In this embodiment, the second latch array 44 has clearance slots 58 which allow the two latch arrays to be interlaced for a more compact assembly. The compact, interlaced latch array assembly 60 is visible in FIG. 2B.

The latch assembly 60 is placed within an inner housing 62, while the ends of the pivot axes 54, 56 pass out holes 64, 66 on either side (only one side is visible in this view) and are held in place (while still remaining pivotable) by bushings 68, 70.

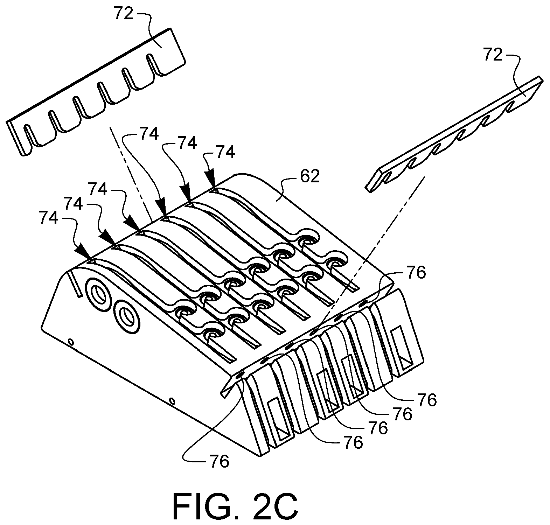

As illustrated in FIG. 2C, optional combs 72 may be provided to interface with the housing and assist with holding tubes (not shown) for guiding suture into the housing 62. Suture will enter through the inner housing 62 in channels 74 from the distal end and exit the inner housing 62 on the proximal end at openings 76.

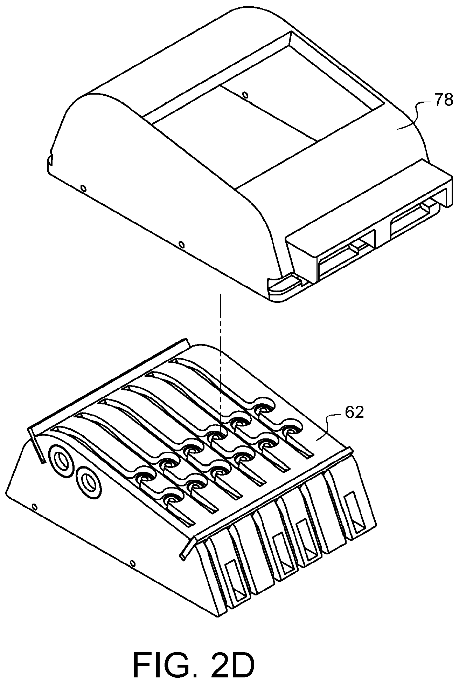

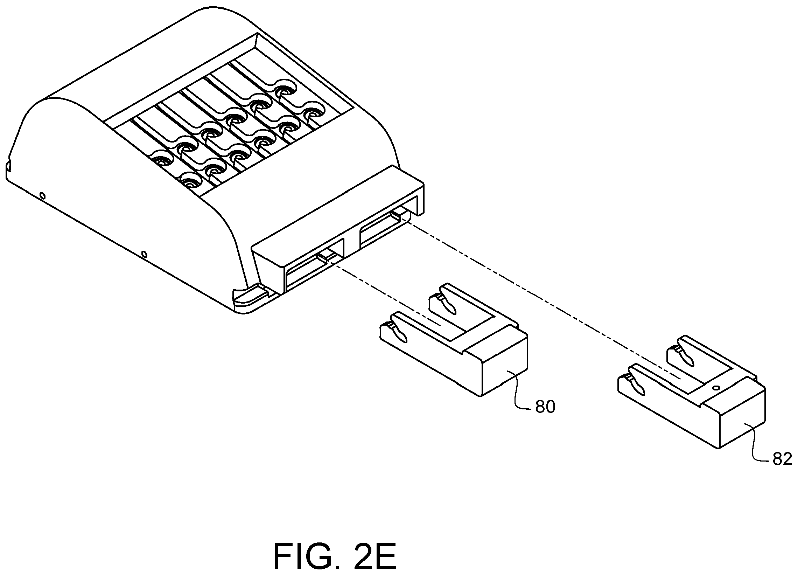

As illustrated in FIG. 2D, an outer housing 78 may be placed over the inner housing 62. As illustrated in FIG. 2E, a first actuator 80 is passed through the outer and inner housings 78, 62 to couple with the cam linkage 50 of the first latch array 42. Similarly, a second actuator 82 is passed through the outer and inner housings 78, 62 to couple with the cam linkage 52 of the second latch array 44.

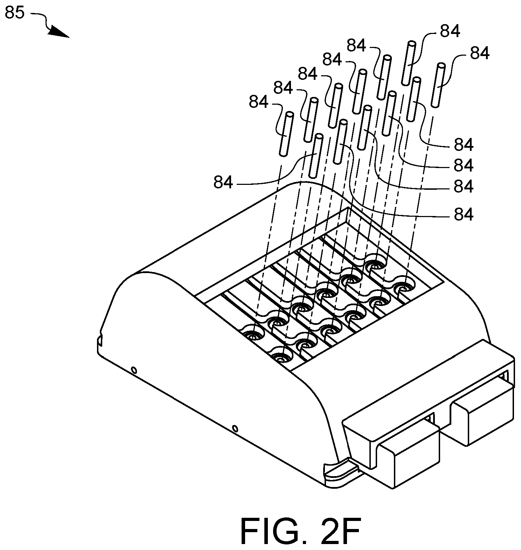

As illustrated in FIG. 2F, temporary assembly posts 84 are placed in alignment holes in each of the latches 46, 48. Individual sutures are routed around each assembly post 84 so that each suture does not run in a straight line.

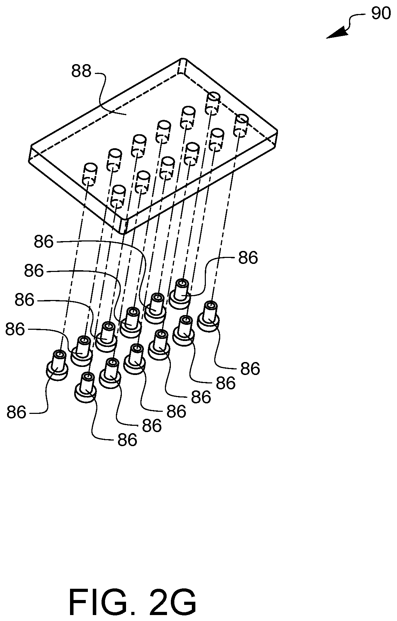

As shown in FIG. 2G, a set of plates 86 are coupled to a transparent window 88 in a pattern which will align with the latches 46, 48 of the previous assemblies. In other embodiments, the plates 86 may be integral with the window, and in still other embodiments, the transparent window may only be translucent or not translucent or transparent at all (in which case the viewing functionality would be lost for such embodiments).

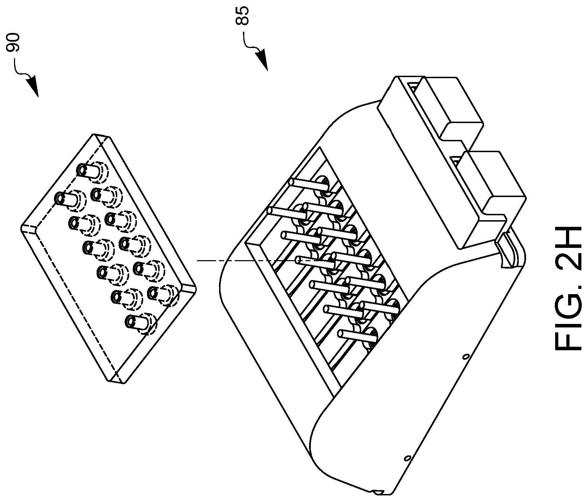

As shown in FIG. 2H, the window and plate assembly 90 from FIG. 2G is placed over the housing assembly 85 from FIG. 2F. Each of the plates 86 has a hole therein which allows the temporary assembly posts to pass through, enabling the plates 86 and the latches 46, 48 to pinch a single suture section therebetween (six held by the first latch array, another six held by the second latch array).

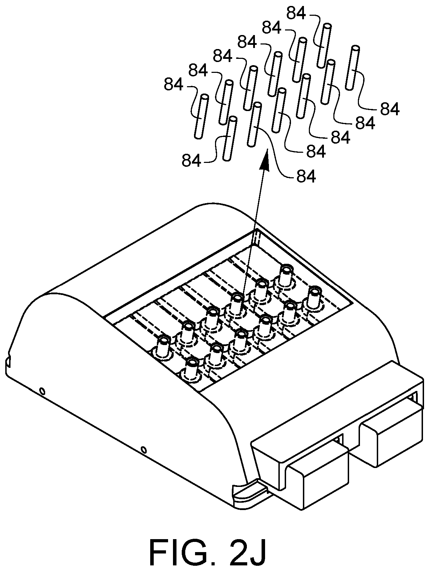

There is not FIG. 2I, as this number looks too similar to the number twenty-one. Then, as shown in FIG. 2J, the temporary assembly posts 84 are removed from the device.

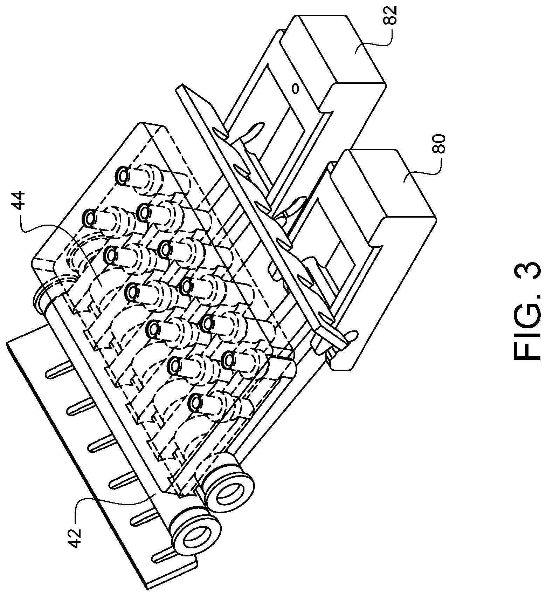

FIG. 3 is a partially exposed view of the assembly of FIG. 2J where the two latch arrays 42, 44 may be seen pressing their latches up against the corresponding plates. The first actuator 80 will release the sutures held by the first latch array 42, while the second actuator will release the sutures held by the second latch array 44.

Various advantages of a suture security device for minimally invasive suturing have been discussed above. Embodiments discussed herein have been described by way of example in this specification. It will be apparent to those skilled in the art that the forgoing detailed disclosure is intended to be presented by way of example only. Various alterations, improvements, and modifications will occur and are intended to those skilled in the art, though not expressly stated herein. These alterations, improvements, and modifications are intended to be suggested hereby.

## Claims

1. A suture security device (20) for automated surgical suturing, comprising:
a latch (22) comprising a cam linkage (38) and being pivotable about an axis (36);
a plate (24);
wherein the latch (22) is configured to be selectively held towards the plate (24), thereby being configured to grip a suture therebetween; and
an actuator (34) coupled to the latch (22) by the cam linkage (38) and operable to move the latch away from the plate (24).

2. The suture security device (20) of claim 1, further comprising a housing (78; 62).

3. The suture security device (20) of claim 2, further comprising a window (40) coupled to the housing (78; 62).

4. The suture security device (20) of claim 3, wherein the window (88) is comprised of a transparent material.

5. The suture security device (20) of claim 1, further comprising one or more suture channels.

6. A suture security device (20) for automated surgical suturing, comprising:
a first latch array (42) comprising a plurality of latches (46) and being pivotable about an axis (54), wherein the first latch array (42) comprises a first cam linkage (50);
a second latch array (44) comprising a plurality of latches (48) and being pivotable about an axis (56), wherein the second latch array (42) comprises a second cam linkage (52);
a plurality of plates (86);
wherein the first latch array (22) and the second latch array (44) are configured to be selectively held towards the plurality of plates (24), thereby being configured to grip a suture therebetween;
a first actuator (80) coupled to the first latch array (42) by the first cam linkage (50) and operable to move the first latch array (42) away from the plurality of plates (86); and
a second actuator (82) coupled to the second latch array (44) by the second cam linkage (52) and operable to move the second latch array (44) away from the plurality of plates (86).

7. The suture security device (20) of claim 6, further comprising one or more combs (72) configured to assist with holding tubes.

8. The suture security device (20) of claim 6, further comprising a housing (78; 62).

9. The suture security device (20) of claim 8, further comprising a window (40) coupled to the housing (78; 62).

10. The suture security device (20) of claim 9, wherein the window (88) is comprised of a transparent material.

11. The suture security device (20) of claim 6, further comprising one or more suture channels.

## Patentansprüche

1. Nahtmaterial-Sicherheitsvorrichtung (20) für automatisiertes chirurgisches Nähen, umfassend:
eine Verriegelung (22), die eine Nockenverbindung (38) umfasst und um eine Achse (36) schwenkbar ist;
eine Platte (24);
wobei die Verriegelung (22) so ausgestaltet ist, dass sie wahlweise in Richtung der Platte (24) gehalten wird, wodurch sie konfiguriert ist, ein Nahtmaterial dazwischen zugreifen; und
ein Betätigungselement (34), das durch die Nockenverbindung (38) mit der Verriegelung (22) gekoppelt ist und betätigt werden kann, um die Verriegelung von der Platte (24) wegzubewegen.

2. Nahtmaterial-Sicherheitsvorrichtung (20) nach Anspruch 1, ferner umfassend ein Gehäuse (78; 62).

3. Nahtmaterial-Sicherheitsvorrichtung (20) nach Anspruch 2, ferner umfassend ein Fenster (40), das mit dem Gehäuse (78; 62) verbunden ist.

4. Nahtmaterial-Sicherheitsvorrichtung (20) nach Anspruch 3, wobei das Fenster (88) aus einem transparenten Material besteht.

5. Nahtmaterial-Sicherheitsvorrichtung (20) nach Anspruch 1, ferner umfassend einen oder mehrere Nahtmaterialkanäle.

6. Nahtmaterial-Sicherheitsvorrichtung (20) für automatisiertes chirurgisches Nähen, umfassend:
eine erste Verriegelungsanordnung (42), die eine Vielzahl von Verriegelungen (46) umfasst und um eine Achse (54) schwenkbar ist, wobei die erste Verriegelungsanordnung (42) eine erste Nockenverbindung (50) umfasst;
eine zweite Verriegelungsanordnung (44), die eine Vielzahl von Verriegelungen (48) umfasst und um eine Achse (56) schwenkbar ist, wobei die zweite Verriegelungsanordnung (42) eine zweite Nockenverbindung (52) umfasst;
eine Vielzahl von Platten (86);
wobei die erste Verriegelungsanordnung (22) und die zweite Verriegelungsanordnung (44) so ausgestaltet sind, dass sie wahlweise in Richtung der Vielzahl von Platten (24) gehalten werden, wodurch sie konfiguriert sind, ein Nahtmaterial dazwischen zugreifen;
einen ersten Aktuator (80), der durch die erste Nockenverbindung (50) mit der ersten Verriegelungsanordnung (42) gekoppelt ist und betreibbar ist, um die erste Verriegelungsanordnung (42) von der Vielzahl von Platten (86) weg zu bewegen; und
einen zweiten Aktuator (82), der mit der zweiten Verriegelungsanordnung (44) durch die zweite Nockenverbindung (52) gekoppelt ist und betreibbar ist, um die zweite Verriegelungsanordnung (44) von der Vielzahl von Platten (86) weg zu bewegen.

7. Nahtmaterial-Sicherheitsvorrichtung (20) nach Anspruch 6, ferner umfassend einen oder mehrere Kämme (72), die so ausgestaltet sind, dass sie beim Halten von Hülsen helfen.

8. Nahtmaterial-Sicherheitsvorrichtung (20) nach Anspruch 6, ferner umfassend ein Gehäuse (78; 62).

9. Nahtmaterial-Sicherheitsvorrichtung (20) nach Anspruch 8, ferner umfassend ein Fenster (40), das mit dem Gehäuse (78; 62) verbunden ist.

10. Nahtmaterial-Sicherheitsvorrichtung (20) nach Anspruch 9, wobei das Fenster (88) aus einem transparenten Material besteht.

11. Nahtmaterial-Sicherheitsvorrichtung (20) nach Anspruch 6, ferner umfassend einen oder mehrere Nahtmaterialkanäle.

## Revendications

1. Un dispositif de sécurité de suture (20) pour la suture chirurgicale automatisée, comprenant :
un verrou (22) comprenant une liaison à came (38) et pouvant pivoter autour d'un axe (36) ;
une plaque (24) ;
dans lequel le verrou (22) est configuré pour être sélectivement maintenu vers la plaque (24), ce qui permet de saisir une suture entre les deux ; et
un actionneur (34) couplé au verrou (22) par la liaison à came (38) et capable d'éloigner le verrou de la plaque (24).

2. Le dispositif de sécurité de suture (20) de revendication 1, comprend en outre un boîtier (78 ; 62).

3. Le dispositif de sécurité de suture (20) de revendication 2, comprend en outre une fenêtre (40) couplée au boîtier (78 ; 62).

4. Le dispositif de sécurité de suture (20) de la revendication 3, dans lequel la fenêtre (88) est constituée d'un matériau transparent.

5. Le dispositif de sécurité de suture (20) de revendication 1, comprend en outre un ou plusieurs canaux de suture.

6. Le dispositif de sécurité de suture (20) pour la suture chirurgicale automatisée, comprenant :
un premier ensemble de verrous (42) comprenant plusieurs verrous (46) et pouvant pivoter autour d'un axe (54), le premier ensemble de verrous (42) comprenant une première liaison à came (50) ;
un deuxième ensemble de verrous (44) comprenant plusieurs verrous (48) et pouvant pivoter autour d'un axe (56), le deuxième ensemble de verrous (42) comprenant une deuxième liaison par came (52) ;
une pluralité de plaques (86) ;
dans lequel le premier ensemble de verrous (22) et le second ensemble de verrous (44) sont configurés pour être sélectivement maintenus vers la pluralité de plaques (24), étant ainsi configurés pour saisir une suture entre eux ;
un premier actionneur (80) couplé au premier ensemble de verrous (42) par la première liaison à came (50) et capable à éloigner le premier ensemble de verrous (42) de la pluralité de plaques (86) ; et
un deuxième actionneur (82) couplé au second ensemble de verrous (44) par la deuxième liaison à came (52) et capable d'éloigner le second ensemble de verrous (44) de la pluralité de plaques (86).

7. Le dispositif de sécurité de suture (20) de revendication 6, comprend en outre un ou plusieurs peignes (72) configurés pour aider à maintenir les tubes.

8. Le dispositif de sécurité de suture (20) de revendication 6, comprend en outre un boîtier (78 ; 62).

9. Le dispositif de sécurité de suture (20) de revendication 8, comprend en outre une fenêtre (40) couplée au boîtier (78 ; 62).

10. Le dispositif de sécurité de suture (20) de revendication 9, dans lequel la fenêtre (88) est constituée d'un matériau transparent.

11. Le dispositif de sécurité de suture (20) de revendication 6, comprend en outre un ou plusieurs canaux de suture.
